# EUROPÄISCHE PATENTSCHRIFT

(11) **EP 2 170 440 B1**
(45) Veröffentlichungstag und Bekanntmachung des Hinweises auf die Patenterteilung: **26.04.2017**
(21) Anmeldenummer: 08785096.2
(22) Anmeldetag: 25.07.2008
(51) Int. Cl.: A61K 9/00, A61M 5/42, A61M 5/32

(54) **INJEKTIONSEINRICHTUNG ZUR INJEKTION IN BIOLOGISCHES GEWEBE UND INJEKTIONSDEPOT**
INJECTION DEVICE FOR INJECTION INTO BIOLOGICAL TISSUE, AND INJECTION DEPOT
DISPOSITIF D'INJECTION POUR L'INJECTION DANS DES TISSUS BIOLOGIQUES ET DÉPÔT PAR INJECTION

(30) Priorität: 25.07.2007 DE 102007034682
(43) Veröffentlichungstag der Anmeldung: 07.04.2010
(73) Patentinhaber: Innovacell Biotechnologie AG, 6020 Innsbruck (AT)
(72) Erfinder: SCHWAIGER, Wolfgang, A-6020 Innsbruck (AT); MARKSTEINER, Rainer, A-6130 Schwaz (AT)
(74) Vertreter: Hertz, Oliver
(86) Internationale Anmeldenummer: PCT/EP2008/006143
(87) Internationale Veröffentlichungsnummer: WO 2009/013011

(56) Entgegenhaltungen:
- WO-A-02/45588
- WO-A-2006/128718
- FR-A- 2 895 681
- US-B1- 6 309 374

## Beschreibung

Die Erfindung betrifft eine Injektionseinrichtung, die zur Positionierung und Betätigung einer Injektionsspritze eingerichtet ist, und ein mit einer derartigen Injektionseinrichtung ausgestattetes medizinisches Gerät. Es werden auch Verfahren zur Injektion einer Injektionssubstanz in biologisches Gewebe, insbesondere in Muskelgewebe eines Menschen, und ein Injektionsdepot in biologischem Gewebe beschrieben.

Es ist bekannt, eine biologisch wirksame Injektionssubstanz in biologisches Gewebe zu injizieren, um beispielsweise eine pharmakologische Wirkung zu erzielen oder ein Wachstum oder eine Zelldifferenzierung im Gewebe zu beeinflussen. Die Injektion erfolgt mit einer Injektionsnadel bei gleichzeitiger Beobachtung des Gewebes zum Beispiel mit einer Ultraschallsonde. Für praktische medizinische Anwendungen ist eine präzise Positionierung der Injektionsnadel von erheblicher Bedeutung, um die Injektionssubstanz genau an einem gewünschten Injektionsort zu deponieren und um die Verletzungsgefahr für die Umgebung des Gewebes zu minimieren.

Zur Positionierung der Injektionsnadel wird eine Injektionseinrichtung mit einer Nadelführung verwendet, die eine vorbestimmte Position und Ausrichtung relativ zu der Ultraschallsonde aufweist. In WO 02/45588 A1 und DE 10 2005 025 639 A1 werden Injektionsgeräte beschrieben, die eine Ultraschallsonde und eine Injektionseinrichtung mit einer Nadelführung aufweisen. Die herkömmlichen Injektionsgeräte sind zur Injektion durch die innere Wand von Körperhöhlen oder -röhren, z. B. in die Harnröhre vorgesehen. Hierzu weist die Nadelführung eine in Bezug auf die axiale Ausrichtung der Ultraschallsonde radial nach außen weisende Krümmung auf. Der Injektionsort im Gewebe wird durch die Krümmung der Nadelführung und die Austrittslänge einer durch die Nadelführung in das Gewebe vorgeschobenen Injektionsnadel bestimmt.

Die Injektion mit den herkömmlichen Injektionsgeräten erfolgt derart, dass die Injektionseinrichtung in Verbindung mit der Ultraschallsonde durch eine Körperöffnung eingeführt und im Körper positioniert wird. Die Injektionsnadel wird bis zu einem vorbestimmten Injektionsort vorgeschoben. In diesem Zustand erfolgt die Injektion der Injektionssubstanz am Injektionsort, wobei ein kugelförmiges Depot im Gewebe gebildet wird. Anschließend wird die Nadel zurückgezogen und die Injektionseinrichtung für eine weitere Injektion an einem anderen Injektionsort neu ausgerichtet. Die Injektion an einer Vielzahl von Injektionsorten insbesondere entlang eines sich longitudinal entlang eines röhrenförmigen Hohlorgans erstreckenden Gewebes erfordert somit eine Vielzahl von Injektionsschritten, bei denen abwechselnd ein neuer Injektionsort eingestellt und die Injektionssubstanz an dem eingestellten Injektionsort deponiert wird. Die Bildung einer Vielzahl von Einstichen kann wegen der Vernarbung und damit einer Destabilisierung des behandelten Hohlorgans von Nachteil sein.

Mit den herkömmlichen Injektionsgeräten wurde zwar ein Fortschritt für die genaue Deposition biologisch wirksamer Substanzen in ein Gewebe erreicht. Nachteilig ist jedoch, dass die Anwendung der herkömmlichen Injektionsgeräte auf die Injektion durch die Innenwand der Körperhöhle oder -röhre beschränkt ist. In der medizinischen Praxis sind die herkömmlichen Injektionsgeräte für eine Injektion durch die äußere Körperoberfläche ungeeignet. Die Injektion durch die Innenwand kann jedoch z. B. aus medizinischen Gründen unerwünscht sein. Beispielsweise hätten die herkömmlichen Injektionsgeräte bei einer Injektion in den Schließmuskel des Enddarms den Nachteil, dass bei einem Durchstechen der Schleimhautschicht (Mukosa) eine hohe Infektionsgefahr bestehen würde. Dieser Nachteil wird noch durch die Beschränkung auf kugelförmige Depots verstärkt, da für eine Injektion an einer Vielzahl von Injektionsorten viele Einstiche durch die Innenwand im Körper notwendig wären.

Aufgrund dieser Nachteile eignen sich die herkömmlichen Injektionsgeräte insbesondere nicht für eine Injektion in den Schließmuskel des Enddarms. Der Schließmuskel des Enddarms umfasst im wesentlichen die Teile Mukosa, interner Sphinkter, externer Sphinkter und Muskel Puborectalis. Der externe Sphinkter geht in den Muskel Puborectalis über, der wiederum mit dem Muskel pubococcygeus und dem Muskel iliococcygeus den Muskel Levator ani (Teil des Beckenbodens) bildet. Die Mukosa ist eine sehr gut durchblutete Schleimhautschicht mit mehreren Gewebetypen, welche die Innenwand des Darms auskleidet und als "Dichtung" des Enddarms dient. Wird diese Schicht beschädigt, kann eine Stuhlinkontinenz die Folge sein (z. B. Hämorridenentfernung). Der interne Schließmuskel (interner Sphinkter) ist ein aus glatten Muskelzellen bestehender Muskel, der rund um den Enddarm angeordnet ist. Der externe Schließmuskel (externer Sphinkter) ist ein aus quergestreiften Muskelzellen bestehender Muskel, der außen um den internen Sphinkter liegt. Beide Schließmuskeln sind wesentlich für die Darmkontinenz, wobei zusätzlich auch der Beckenboden und der anorektale Winkel (Winkel zwischen Enddarm/Dickdarm) eine Funktion für den Schließmechanismus erfüllen.

Durch Überdehnung oder chirurgische Eingriffe kann es zu Verletzungen der oben genannten Muskelstränge kommen, so dass in der Folge Stuhlinkontinenz auftreten kann. Beispielsweise hat der externe Schließmuskel einen Bereich verringerter Dicke, an dem insbesondere bei Frauen durch eine Geburt Schäden verursacht werden können. Als Folge vernarbt der Muskel, wobei er jedoch seine Funktion nicht mehr übernehmen kann, so dass Stuhlinkontinenz auftritt.

Herkömmliche Methoden zur Behandlung der Stuhlinkontinenz basieren auf Beckenbodenübungen (konservativ) oder auf einer Überlappungsoperation, bei der Bruchstellen der Schließmuskels überlappt und vernäht werden oder ein künstlicher Schließmuskel implantiert wird. Oft reicht die konservative Behandlung jedoch nicht mehr aus, während die operativen Eingriffe riskant und unzuverlässig sind.

Aus der Praxis sind Füllstofftherapien bekannt, bei denen in Muskeln Füllstoffe injiziert werden (sog. "bulk injection"). Diese Füllstöfftherapien haben jedoch den Nachteil, dass das Muskelgewebe durch einen inneren Druck des Füllstoffs aufgespannt wird und dadurch beschädigt werden kann.

Aus FR 2 895 681 A ist ein Injektionsgerät zur Injektion von Flüssigkeiten oder Pasten in Hautgewebe bekannt, bei dem eine Injektion während einer Rückzugsbewegung einer Injektionsnadel erfolgen kann. Ein weiteres Injektionsgerät zur Injektion einer Flüssigkeit in biologisches Gewebe ist aus US 6 309 374 B1 bekannt.

Die Aufgabe der Erfindung ist es, eine verbesserte Injektionseinrichtung für eine Injektionsspritze bereitzustellen, mit der die Nachteile und Beschränkungen der herkömmlichen Techniken überwunden werden und die insbesondere zur Injektion in einen Schließmuskel des Enddarms geeignet ist. Die Aufgabe der Erfindung ist es auch, ein verbessertes medizinisches Gerät, das eine derartige Injektionseinrichtung aufweist, bereitzustellen.

Diese Aufgaben werden durch eine Injektionseinrichtung und ein medizinisches Gerät mit den Merkmalen der unabhängigen Ansprüche gelöst. Vorteilhafte Ausführungsformen und Anwendungen der Erfindung ergeben sich aus den abhängigen Ansprüchen.

Vorrichtungsbezogen wird die Aufgabe gemäß einem ersten Gesichtspunkt der Erfindung insbesondere durch die technische Lehre gelöst, eine Injektionseinrichtung mit einer Führungseinrichtung, in die eine Injektionsspritze einsetzbar ist, und einer Betätigungseinrichtung bereitzustellen, die mit einer in der Führungseinrichtung eingesetzten Injektionsspritze so zusammenwirkt, dass bei einer Rückzugbewegung der Injektionsspritze ein Ausstoß einer Injektionsflüssigkeit erfolgt. Die Betätigungseinrichtung weist ein Betätigungselement auf, das einen Anschlag für eine Kolbeneinrichtung der Injektionsspritze bildet. Das Betätigungselement ist relativ zu der Führungseinrichtung ortsfest oder mit einer Geschwindigkeit verschiebbar angeordnet, die von der Geschwindigkeit der Rückzugbewegung abweicht. Vorteilhafterweise ermöglicht die erfindungsgemäße Injektionseinrichtung, dass durch die Rückzugbewegung der Injektionsspritze, insbesondere einer Injektionsnadel und eines Spritzenkörpers die Injektion einer im Spritzenkörper enthaltenen Injektionssubstanz bewirkt wird. Die Kolbeneinrichtung führt während der Rückzugbewegung eine zu der Injektionsnadel und dem Spritzenkörper entgegengesetzte Relativbewegung aus. Vorteilhafterweise ermöglicht die Erfindung eine schonende Injektion in den von der Injektionsnadel beim Rückzug im Gewebe geschaffenen Raum. Die entlang einer Injektionsbahn deponierten Zellen bleiben lebensfähig, so dass ein effektives Anwachsen im Gewebe ermöglicht wird.

Gemäß einem zweiten Gesichtspunkt wird die oben genannte Aufgabe der Erfindung durch die allgemeine technische Lehre gelöst, ein medizinisches Gerät bereitzustellen, das die erfindungsgemäße Injektionseinrichtung und eine in diese eingesetzte Injektionsspritze umfasst. Vorteilhafterweise hat das erfindungsgemäße medizinische Gerät eine im Vergleich zu den herkömmlichen Injektionsgeräten neue Funktionalität, da mit der Injektionsspritze gleichzeitig zu der Rückzugbewegung zuverlässig linienförmige Injektionsdepots gebildet werden können.

Mit dem Begriff "Injektionsspritze" wird hier allgemein jede Flüssigkeitsfördereinrichtung mit einem Flüssigkeitsreservoir (Spritzenkörper mit mindestens einer Kammer), einer Injektionskanüle (hohle Spritzennadel) und einer Kolbeneinrichtung mit mindestens einem Spritzenkolben und mindestens einer Schubstange bezeichnet. Die Spritzennadel kann gerade oder gekrümmt sein. Der Spritzenkörper kann eine oder mehrere Kammern umfassen, die vorzugsweise eine Zylinderform haben. Jede der Kammern ist zur Aufnahme eines Spritzenkolbens vorgesehen. Durch eine Verschiebung des mindestens einen Spritzenkolbens in dem Spritzenkörper kann Flüssigkeit aus dem Spritzenkörper durch die Spritzennadel ausgestoßen werden. Die Injektionsspritze kann z. B. einen Spritzenkörper mit einer einzigen Kammer, mit der die Spritzennadel verbunden und in der ein Spritzenkolben angeordnet ist, oder zwei oder mehr Kammern umfassen, mit denen die Spritzennadel verbunden und in denen jeweils ein Spritzenkolben angeordnet ist. Erfindungsgemäß kann die Spritzennadel zusätzlich als Elektromyographie-Sonde (EMG-Sonde) verwendet werden, um elektrische Signale aus dem Gewebe, in das die Injektion erfolgt, ableiten zu können.

Mit dem Begriff "Injektionssubstanz" wird hier allgemein jede Flüssigkeit bezeichnet, die mindestens eine pharmakologisch oder biologisch wirksame Substanz in gelöster oder suspendierter Form enthält. Gemäß einer bevorzugten Anwendung der Erfindung umfasst die Injektionssubstanz eine Zellsupension, insbesondere eine Myoblastensuspension.

Es wird auch beschrieben, eine Injektionssubstanz unter Verwendung der erfindungsgemäßen Injektionseinrichtung und einer Injektionsspritze in einem Organismus zu deponieren, indem die Injektion der Injektionssubstanz während einer Rückzugbewegung der Injektionsspritze aus dem Organismus erfolgt. Dabei werden vorzugsweise zunächst die Führungseinrichtung relativ zu dem Organismus ausgerichtet, anschließend die Injektionsspritze in die Führungseinrichtung eingesetzt und in den Organismus vorgeschoben und schließlich der Spritzenkörper und die Injektionsnadel der Injektionsspritze gemeinsam zurückgezogen, wobei während des Rückzugs mit dem Betätigungselement der erfindungsgemäßen Injektionseinrichtung der Spritzenkolben der Injektionsspritze betätigt wird.

Vorteilhafterweise wird erfindungsgemäß ein neues Depositionsprinzip realisiert, indem die Injektionssubstanz in einem von der Spritzennadel im Organismus gebildeten Injektionskanal deponiert wird. Die Betätigungseinrichtung mit dem ausschließlich während der Rückzugbewegung wirkenden Betätigungselement ermöglicht abweichend von der herkömmlichen Erzeugung kugelförmiger Depots die Bildung linienförmiger Injektionen. Es können mit hoher Effektivität nach einer einmaligen Positionierung der Injektionsnadel im Gewebe und einem einmaligen Einstich größere Mengen der Injektionssubstanz im Gewebe deponiert werden.

Es werden auch beschrieben: ein linienförmig gebildetes Injektionsdepot (Zellimplantat), insbesondere aus autologen Myoblasten, z. B. mit autologem Serum, in einem biologischen Gewebe; die Verwendung von autologen Myoblasten, z. B. mit autologem Serum, in Gestalt eines linienförmigen Injektionsvolumens, das in einem Schließmuskel des Enddarms angeordnet ist, zur Behandlung der Stuhlinkontinenz; die Verwendung von autologen Myoblasten, z. B. mit autologem Serum, zur Herstellung eines linienförmigen Injektionsvolumens, das in einem Schließmuskel des Enddarms angeordnet ist, zur Behandlung der Stuhlinkontinenz; und ein Verfahren zur Herstellung eines Arzneimittels zur Behandlung der Stuhlinkontinenz, bei dem als Bestandteil des Arzneimittels autologen Myoblasten, z. B. mit autologem Serum, verwendet wird. Die Begriffe "linienförmiges Injektionsvolumen" oder "linienförmiges Injektionsdepot" bezeichnen allgemein eine bahn-, band- oder fadenförmige Substanzanordnung (Zellimplantat) mit einer Längsdimension, die größer als die Querdimension der Substanzanordnung ist.

Die Bildung eines linienförmigen Zellimplantats mit lebenden Zellen hat den besonderen Vorteil einer gleichmäßigen Verteilung der Zellen im Gewebe, was zu einem besonders guten Anwachsen der Zellen führt.

Gemäß einer bevorzugten Ausführungsform der erfindungsgemäßen Injektionseinrichtung ist das Betätigungselement so angeordnet, dass der Spritzenkolben während der Vorschubbewegung der Injektionsnadel und des Spritzenkörpers frei beweglich ist. Vorteilhafterweise kann dadurch das Einsetzen der Injektionsspritze in die Injektionseinrichtung erleichtert werden. Vorzugsweise ist die Betätigungseinrichtung mit einer Stelleinrichtung ausgestattet, über die das Betätigungselement mit der Führungseinrichtung verbunden ist.

Die Stelleinrichtung kann vorteilhafterweise mehrere Funktionen erfüllen. Beispielsweise kann die Stelleinrichtung zur Bewegung des Betätigungselements zwischen einer Freigabeposition, in der der Spritzenkolben während der Vorschubbewegung der Injektionsspritze frei beweglich ist, und einer Anschlagposition bewegt werden, in der der Spritzenkolben während der Rückzugbewegung betätigt wird. Hierzu weist die Stelleinrichtung beispielsweise einen Schwenkhebel auf, mit dem das Betätigungselement relativ zu der Führungseinrichtung verschwenkt werden kann. Mit dem Schwenkhebel kann das Betätigungselement zwischen den genannten Freigabe- und Anschlagpositionen bewegt werden.

Des weiteren kann die Stelleinrichtung ein Translationselement enthalten, mit dem das Betätigungselement in der Anschlagposition relativ zu der Führungseinrichtung verschiebbar ist. Mit dem Translationselement kann die Position des Betätigungselements an die Größe und Bauform der konkret verwendeten Injektionsspritze angepasst werden. Des Weiteren kann das Betätigungselement mit dem Translationselement während der Rückzugbewegung der Injektionsspritze verschoben werden. Es ist insbesondere eine Geschwindigkeit des Translationselements einstellbar, die geringer als die Geschwindigkeit der Rückzugbewegung oder zu dieser entgegengesetzt ist. Hierzu ist das Translationselement vorzugsweise mit einem Übertragungsmechanismus ausgestattet, mit dem die Geschwindigkeit des Betätigungselements relativ zur Geschwindigkeit der Injektionsnadel und des Spritzenkörpers einstellbar ist. Alternativ oder zusätzlich kann das Translationselement für die Verschiebung des Betätigungselements zwischen den Freigabe- und Anschlagpositionen vorgesehen sein.

Gemäß einer weiteren vorteilhaften Variante der Erfindung kann das Translationselement mit einem Motorantrieb ausgestattet sein, mit dem das Betätigungselement relativ zu der Führungseinrichtung verschiebbar ist. In diesem Fall kann vorteilhafterweise die Betätigung des Spritzenkolbens unabhängig von der Geschwindigkeit der Rückzugbewegung der Injektionsnadel und des Spritzenkörpers eingestellt werden.

Gemäß der Erfindung ist die Führungseinrichtung zur Einstellung eines Injektionswinkels der Injektionsspritze eingerichtet. Der Injektionswinkel (Einstichwinkel, Neigungswinkel) ist der Winkel zwischen den longitudinalen Richtung eines Hohlorgans, z. B. des Enddarms, in dessen Wand eine Injektionssubstanz eingeführt werden soll, und der Injektionsnadel, insbesondere der Winkel zwischen der longitudinalen Richtung einer bildgebenden Sonde, die in das Hohlorgans eingeführt wird, und der Injektionsnadel. Erfindungsgemäß ist der Injektionswinkel vorzugsweise so gewählt, dass die Injektionsnadel parallel zu der longitudinalen Richtung oder radial nach innen, d. h. zu der Mitte des Hohlorgans, insbesondere der bildgebenden Sonde weist. Dies ermöglicht die sichere Injektion von der Außenseite des Organismus her in die Wand des Hohlorgans.

Gemäß der Erfindung umfasst die Führungseinrichtung der Injektionseinrichtung ein Trägerteil, das mit einem verschwenkbaren Führungsrohr zur Aufnahme der Injektionsnadel ausgestattet ist. Mit dem Führungsrohr kann der Injektionswinkel der Injektionsspritze einstellbar sein. Vorteilhafterweise ist das Führungsrohr für eine stabile Aufnahme der Injektionsspritze an der Führungseinrichtung ausreichend. Durch die Verschwenkung des Führungsrohrs kann der Injektionswinkel der Injektionsnadel in einem Organismus, auf den die Injektionseinrichtung mit der Führungseinrichtung aufgesetzt ist, gewählt werden. Vorteilhafterweise kann mit dem Injektionswinkel auch die Einstichhöhe im Gewebe variabel eingestellt werden.

Vorzugsweise ist das Führungsrohr in der Führungseinrichtung mit einem Drehlager und einem Translationslager positioniert, wobei der Injektionswinkel der Injektionsnadel durch eine Verschiebung des Translationslagers relativ zu dem sich frei mitdrehenden Drehlager einstellbar ist.

Die Stabilität der Aufnahme der Injektionsspritze an der Führungseinrichtung kann gemäß einer Variante der Erfindung vorteilhafterweise verbessert werden, wenn ein Schwenkhebel der Betätigungseinrichtung einen Träger für die Injektionsspritze bildet. Der Schwenkhebel und das Führungsrohr sind bei dieser Ausführungsform der Erfindung so ausgerichtet, dass, wenn die Spritzennadel im Führungsrohr angeordnet ist, der Spritzenkörper auf dem Schwenkhebel aufliegt. Durch eine Verschwenkung des Schwenkhebels ist der Injektionswinkel der Spritzennadel einstellbar.

Weitere Vorteile können sich ergeben, wenn die Führungseinrichtung mit einer Halteplatte verbunden ist. Eine Frontseite der Halteplatte ist auf den Organismus aufsetzbar, während die Führungseinrichtung auf einer Rückseite der Halteplatte befestigt ist. Die Halteplatte enthält mindestens eine Durchgangsöffnung, durch welche die Injektionsnadel von der Führungseinrichtung zum Organismus hindurch treten kann. Vorteilhafterweise wird mit der Halteplatte die Ausrichtung der Führungseinrichtung relativ zum Organismus erleichtert. Mit einer ebenen Erstreckungsrichtung der Halteplatte wird eine vorbestimmte radiale Bezugsebene definiert. Die in die Führungseinrichtung eingesetzte Injektionsspritze ist in einer axialen Bezugsrichtung (senkrecht zu der radialen Bezugsebene) oder mit dem vorbestimmten Injektionswinkel relativ zu der axialen Bezugsrichtung verschiebbar.

Für die Einstellung verschiedener Injektionsorte kann es von Vorteil sein, wenn die Halteplatte ein bewegliches, insbesondere drehbares Teil einer ortsfesten Halteeinrichtung ist.

Alternativ oder zusätzlich können an der Halteplatte eine Vielzahl von Durchtrittsöffnungen gebildet sein, die jeweils zur Ausrichtung der Injektionsnadel in Bezug auf einen bestimmten Injektionsort vorgesehen sind.

Gemäß der Erfindung ist die Injektionseinrichtung mit einer bildgebenden Sonde, insbesondere einer Ultraschallsonde, ausgestattet. Die Führungseinrichtung kann auf der bildgebenden Sonde verschiebbar angeordnet sein. Besonders bevorzugt ist die bildgebende Sonde verschiebbar an der Halteplatte angeordnet. Hierzu weist die Halteplatte insbesondere eine Sondenaufnahme auf, in welche die bildgebende Sonde einsetzbar ist.

Weitere Einzelheiten und Vorteile der Erfindung werden aus der nachfolgenden Beschreibung von bevorzugten Ausführungsformen der Erfindung und den beigefügten Zeichnungen ersichtlich. Es zeigen:
- Figur 1:: eine schematische Seitenansicht einer ersten Ausführungsform der erfindungsgemäßen Injektionseinrichtung mit einer Injektionsspritze;
- Figur 2:: eine vergrößerte Ansicht der in Figur 1 gezeigten Führungseinrichtung;
- Figur 3:: eine schematische Vorderansicht der in Figur 1 gezeigten Ausführungsform der erfindungsgemäßen Injektionseinrichtung;
- Figur 4:: eine schematische Seitenansicht einer weiteren Ausführungsformen der erfindungsgemäßen Injektionseinrichtung;
- Figuren 5 und 6:: schematische Seitenansichten von Varianten der in Figur 4 gezeigten Injektionseinrichtung;
- Figur 7:: eine schematische Seitenansicht einer weiteren Ausführungsformen der erfindungsgemäßen Injektionseinrichtung;
- Figur 8:: eine schematische Vorderansicht der in Figur 7 gezeigten Ausführungsform der erfindungsgemäßen Injektionseinrichtung;
- Figur 9:: eine schematische Schnittansicht einer Ausführungsform eines erfindungsgemäß verwendeten Übertragungsmechanismus; und
- Figur 10:: eine schematische Illustration einer Führungseinrichtung für eine gekrümmte Injektionsnadel.

Ausführungsformen der Erfindung werden im folgenden unter beispielhaftem Bezug auf die Injektion mit einer Injektionsspritze erläutert, die einen Spritzenkörper mit einer Kammer und eine Kolbeneinrichtung mit einem Spritzenkolben aufweist. Die praktische Umsetzung der Erfindung ist nicht auf diese Anwendung beschränkt, sondern entsprechend auch mit einer Injektionsspritze möglich, die zwei oder mehr Spritzenkolben in zwei oder mehr Kammern umfasst, deren Inhalte bei der Injektion vermischt werden.

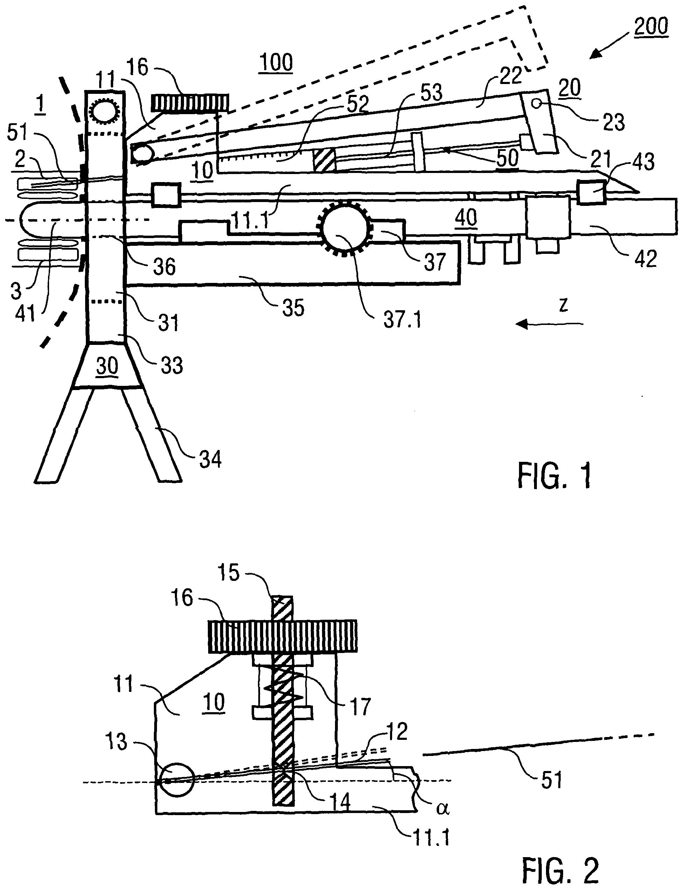

Die in Figur 1 gezeigte erste Ausführungsform der erfindungsgemäßen Injektionseinrichtung 100 umfasst die Führungseinrichtung 10 mit einem Trägerteil 11 und einem Trägerarm 11.1, die Betätigungseinrichtung 20 mit dem Betätigungselement 21 und dem Schwenkhebel 22, die Halteeinrichtung 30 mit der Halteplatte 31, einem Plattenrahmen 33, einem Stativ 34 und einem Haltearm 35, und die Ultraschallsonde 40 mit einem Ultraschallkopf 41 und einem Schaft 42. Das erfindungsgemäße medizinische Gerät 200 wird durch die Kombination der Injektionseinrichtung 100 mit der in die Führungseinrichtung 10 eingesetzten Injektionsspritze 50 gebildet, welche die Injektionsnadel 51, den Spritzenkörper 52 und die Kolbeneinrichtung 53 (Spritzenkolben 53) umfasst. Die Injektionsspritze 50 hat ein Injektionsvolumen von z. B. 1 ml. Beim dargestellten Beispiel wird die radiale Bezugsebene der Halteplatte 31 als x-y-Ebene bezeichnet. Die axiale Bezugsrichtung der Injektionseinrichtung 100 und insbesondere der Ultraschallsonde 40 verläuft in z-Richtung. Die Ultraschallsonde 40 ist in an sich bekannter Weise mit einem Ultraschallgerät (nicht dargestellt) verbunden, mit dem Bilder des Gewebes erfasst und angezeigt werden können.

Figur 1 zeigt auch die Ausrichtung der Injektionseinrichtung 100 relativ zu einem Organismus 1, insbesondere dem externen Schließmuskel 2 des Enddarms 3 (schematisch, nicht maßstabgerecht gezeichnet). Die axiale Länge der Schließmuskeln beträgt z. B. rd. 2.5 cm. Die radialen Dicken der inneren und äußeren Schließmuskel betragen z. B. rd. 2 mm bzw. rd. 5 mm.

Das Trägerteil 11 der Führungseinrichtung 10 bildet eine Aufnahme für die Injektionsspritze 50, insbesondere die Injektionsnadel 51. Weitere Einzelheiten des Trägerteils 11 sind schematisch in Figur 2 illustriert. Das Trägerteil 11 ist ein Bauteil, in dem ein Führungsrohr 12 angeordnet ist. Das Trägerteil 11 ist z. B. aus Kunststoff hergestellt. Das Führungsrohr 12 ist ein Metallrohr, z. B. aus Edelstahl oder Messing, mit einem Innendurchmesser, der für eine formschlüssige Aufnahme der Injektionsnadel 51 gewählt ist. Das Führungsrohr 12 erfüllt eine Doppelfunktion, indem es den Injektionswinkel α (Neigungswinkel der Injektionsnadel 51 relativ zur axialen Bezugsrichtung) festlegt und eine Führung für die Verschiebung der Injektionsspritze bildet. Der Injektionswinkel α bildet ausgehend von der axialen Bezugsrichtung einen spitzen Winkel, vorzugsweise im Bereich von 0° bis 45 °. Für die sichere Führung der Injektionsspritze weist das Führungsrohr 12 vorzugsweise eine Länge im Bereich von 5 mm bis 8 cm auf. Die Länge des Führungsrohrs 12 wird des Weiteren gewählt, um eine bestimmte maximale Einstichtiefe der Injektionsnadel 51 sicherzustellen.

Die Einstichtiefe der Injektionsnadel 51 wird in Abhängigkeit von der gewünschten Anwendung der erfindungsgemäßen Injektionseinrichtung gewählt. Vorteilhafterweise sind große Einstichtiefen möglich, wobei eine Injektion bis in den Muskel Puborectalis ermöglicht wird.

Mit dem Injektionswinkel α wird auch die Einstichhöhe im Gewebe eingestellt. Der Aufbau gemäß Figur 2 ist beispielsweise dazu eingerichtet, durch eine Verschiebung des Translationslagers die radiale Einstichhöhe im Gewebe über einen Bereich von 6 mm zu variieren.

Das Führungsrohr 12 ist relativ zu der axialen Bezugsrichtung (z-Richtung) verschwenkbar. Hierzu ist das Führungsrohr 12 mit einem Drehlager 13 (Kugelgelenk) und einem Translationslager 14 gelagert. Das Drehlager 13 ist an einer vorderen, zur Halteplatte 31 in Injektionsrichtung weisenden Seite des Trägerteils 11 angeordnet und für eine Drehung um eine Achse senkrecht zur axialen Bezugsrichtung eingerichtet. Das Translationslager 14 ist mit Abstand vom Drehlager 13 in einem Gewindestab 15 gebildet. Der Gewindestab 15 kann durch Drehung einer Stellschraube 16 an einer oberen Seite des Trägerteils 11 in radialer Richtung verschoben werden, um den Neigungswinkel des Führungsrohrs 12 einzustellen. Für eine Bewegung radial nach außen wird der Gewindestab 15 aus dem Trägerteil 11 herausgeschoben. Die entgegengesetzte Bewegung radial nach innen wird durch eine Rückstellfeder 17 im Trägerteil 11 unterstützt. Abweichend von der Illustration in Figur 2 kann die Stellschraube 16 in der Mitte oder an einer unteren Seite des Trägerteils 11 vorgesehen sein.

Das Betätigungselement 21 der Betätigungseinrichtung 20 ist über den Schwenkhebel 22 verschwenkbar am Trägerteil 11 angeordnet (Figur 1). Das Betätigungselement 21 und der Schwenkhebel 22 sind z. B. aus Kunststoff hergestellt. Die Betätigungseinrichtung 20 ist in radialer Richtung zwischen einer Freigabeposition (gestrichelt gezeichnet) und einer Anschlagposition (durchgezogen gezeichnet) verschwenkbar. In der Freigabeposition kann die mit einer Injektionssubstanz beladene Injektionsspritze 50, in der somit der Injektionskolben 53 im Injektionskörper zurückgezogen ist, in die Führungseinrichtung 10 eingesetzt werden. Anschließend kann die Betätigungseinrichtung 20 in die Anschlagposition verschwenkt werden. In dieser liegt das Betätigungselement 21 am freien Ende des Injektionskolbens 53 an.

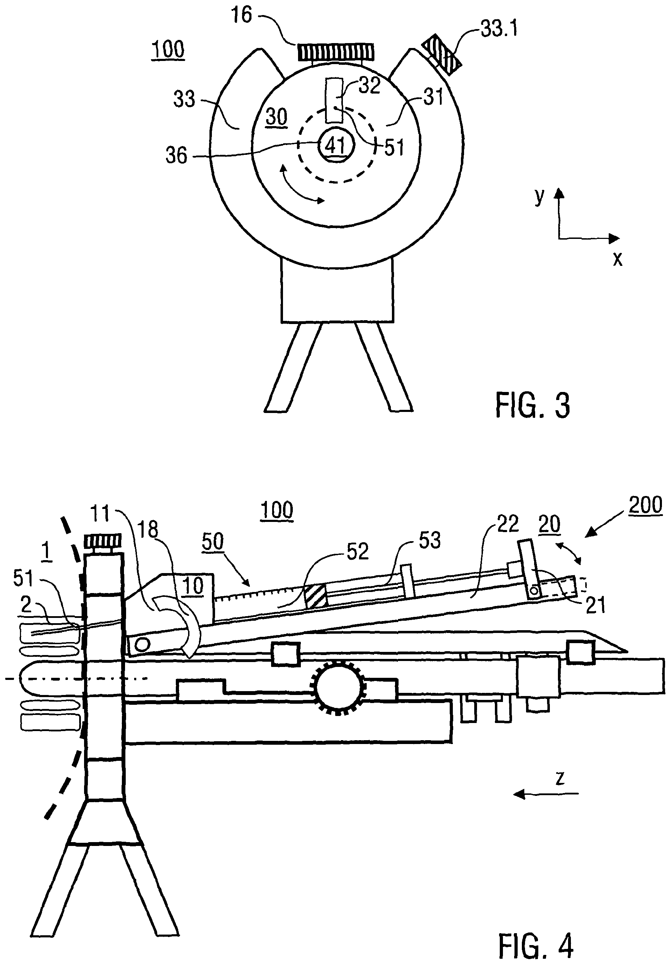

Das Betätigungselement 21 bildet ein Widerlager für den Injektionskolben 53. Alternativ oder zusätzlich zu der Verschwenkbarkeit des Schwenkhebels 22 am Trägerteil 11 kann das Betätigungselement 21 am Schwenkhebel 22 verschwenkbar sein, um die Ausrichtung des Betätigungselements 21 relativ zur eingesetzten Injektionsspritze 50 zu vereinfachen (siehe Figur 4). Das Betätigungselement 21 ist in diesem Fall relativ zum Schwenkhebel 22 mit einer Fixierschraube 23 fixierbar.

Die Ultraschallsonde 40 ist auf dem Haltearm 35 der Halteeinrichtung 30 so angeordnet, dass sich der Ultraschallkopf 41 und der Schaft 42 in der axialen Bezugsrichtung erstrecken. Die Ultraschallsonde 40 ist eine transrektale Sonde, insbesondere mit einem Schaftdurchmesser im Bereich von 1 bis 2 cm, z. B. vom Typ 8818 von B-K Medical. Der Ultraschallkopf 41 ist vorzugsweise für eine Abbildung entlang einer transversalen Schallebene (parallel zur x-y-Ebene) und entlang einer longitudinalen Schallebene (in radialer Richtung) eingerichtet. Die Ultraschallsonde 40 ist auf dem Haltearm 35 in axialer Richtung verschiebbar angeordnet. Hierzu ist bspw. ein Schlitten 37 vorgesehen, mit dem die Ultraschallsonde 40 fest verbunden ist und der auf einer Schiene (nicht dargestellt) im Haltearm 35 verschiebbar ist. Durch Betätigung einer Schlittenschraube 37.1 kann die Ultraschallsonde 40 in axialer Richtung verschoben und/oder fixiert werden.

In Figur 2 ist die Vorderansicht der Injektionseinrichtung 100, insbesondere der Halteeinrichtung 30 gezeigt. Die Halteplatte 31 hat eine Frontseite, die auf den Organismus 1 aufgesetzt wird, und eine Rückseite, an der die Führungseinrichtung 10 befestigt ist. In der Halteplatte 31 sind eine Durchgangsöffnung 32, durch die die Injektionsnadel 51 hindurch tritt, und eine Sondenaufnahme 36 vorgesehen. Die Halteplatte 31 ist im Plattenrahmen 33 drehbar angeordnet. Mit der Halteplatte 31 kann die Führungseinrichtung 10 um die Längsachse der Ultraschallsonde 40 gedreht werden. Durch die Drehung der Halteplatte 31 kann die azimutale Position der Injektionsnadel 51 relativ zum Organismus eingestellt und mit einer Rahmenschraube 33.1 fixiert werden.

Die Führungseinrichtung 10 kann mit der Rückseite der Halteplatte 31 fest verbunden sein. In diesem Fall ist keine feste Verbindung des Trägerarms 12 mit der Ultraschallsonde 40, sondern lediglich eine verschiebbare Auflage 43 vorgesehen. Alternativ kann die Führungseinrichtung 10 ausschließlich über die Ultraschallsonde 40 und den Haltearm 35 mit der Halteplatte 31 verbunden sein. In diesem Fall ist auf der Ultraschallsonde 40 eine Verschiebeeinrichtung (nicht dargestellt) zur Verschiebung und Fixierung der Führungseinrichtung 10 auf der Ultraschallsonde 40 vorgesehen. Die Schiebeeinrichtung kann bspw. eine Kombination aus einem Schlitten und Führungsschienen umfassen.

Die Injektion einer Injektionssubstanz in den Organismus 1, insbesondere den externen Schließmuskel 2 des Enddarms 3 (schematisch, nicht maßstabgerecht gezeichnet) umfasst die folgenden Schritte. Zunächst wird bei einem Untersuchungsschritt die Halteplatte 31 auf den Organismus 1 aufgesetzt. Die Ultraschallsonde 40 wird durch die Halteplatte 30 in den Anus eingeführt und relativ zur Halteplatte 31 fixiert. Mit der Ultraschallsonde 40 wird das Schließmuskelgewebe untersucht, um die Position und Ausrichtung des zu behandelnden Defekts und damit den gewünschten Injektionsort im Schließmuskel festzustellen.

Anschließend folgt ein Kalibrierungsschritt mit einer Justierung der Führungseinrichtung 10. Die Führungseinrichtung 10 wird relativ zur Halteplatte 31 so ausgerichtet, dass die Injektionsnadel einer in die Führungseinrichtung 10 eingesetzten Injektionsspritze am Injektionsort münden würde. Für die Kalibrierung wird die Injektionseinrichtung 100 mit der eingesetzten Injektionsspritze und der Ultraschallsonde 40 in ein Wasserbad getaucht.

Für die folgende Injektion werden zuerst die Halteeinrichtung 30 (ohne die Injektionsspritze) und der Organismus 1 relativ zueinander positioniert. Die Ultraschallsonde 40 wird in den Anus eingeführt. Die Halteplatte 31 wird so gedreht, dass die Führungseinrichtung 10, insbesondere das Führungsrohr 12 auf den gewünschten Injektionsort gerichtet ist. Anschließend wird die Injektionsspritze 50 in die Führungseinrichtung 10 eingesetzt und vorgeschoben, bis die Injektionsnadel 51 den Injektionsort im Gewebe erreicht. Durch das Einstechen der Injektionsnadel 51 in das Gewebe wird ein Injektionskanal (Stichkanal) aufgespannt, der bei der anschließenden Injektion mit der Injektionssubstanz gefüllt wird. Die Injektionsnadel 51 wird unter Ultraschallkontrolle bis zum Injektionsort vorgeschoben, an dem die Zufuhr der Injektionssubstanz beginnen soll. Im Ultraschallgerät werden während der Einführung der Injektionsnadel 51 Bilder des Gewebes erfasst und angezeigt, wobei ggf. Punktionshilfslinien bei der Anzeige der Bilder die Einstellung erleichtern können.

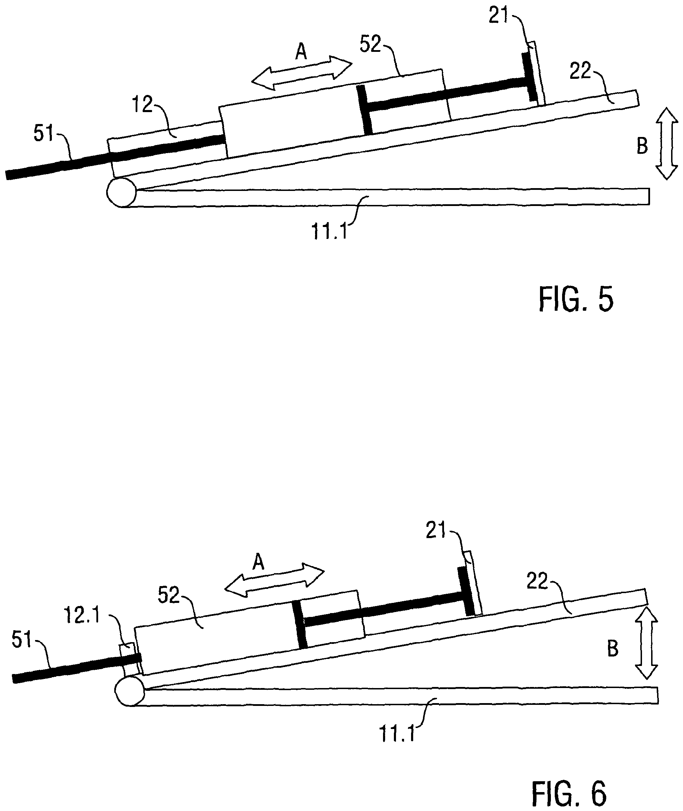

In einem nächsten Schritt wird das Betätigungselement 21 relativ zum Spritzenkolben 53 ausgerichtet. Der Schwenkhebel 22 wird an die Injektionsspritze 50 angelegt und das Betätigungselement 21 als Widerlager zum Spritzenkolben 53 fixiert. Anschließend erfolgt die Injektion, indem die Injektionsspritze 50, insbesondere die Injektionsnadel 51 und der Spritzenkörper 52 zurückgezogen werden, wobei der Injektionskolben 53 festgehalten wird. Im Ergebnis führt der Injektionskolben 53 relativ zur Injektionsnadel 51 und dem Spritzenkörper 52 eine entgegengesetzte Bewegung aus, so dass durch den Rückzug der Injektionsspritze 50 die Injektion in das Gewebe bewirkt wird. Gleichzeitig mit der Rückzugbewegung wird der Injektionskanal gefüllt. Im Gewebe wird ein linienförmiges Injektionsvolumen gebildet. Die Injektion erfolgt in Bahnen - d.h. die Injektionsnadel 51 wird zum tiefsten Punkt im Gewebe geführt und anschließend zurückgezogen. Bei diesem Rückweg wird eine Zellbahn appliziert. Die Rückzugbewegung des Spritzenkörpers 52 mit der Injektionsnadel 51 kann manuell insbesondere durch Ziehen am Spritzenkörper, mit einem Stellrad (nicht dargestellt) oder mit einem an der Führungseinrichtung 10 angeordnet Motorantrieb (siehe auch Figur 6) bewirkt werden. Nach der Injektion wird die Injektionseinrichtung neu ausgerichtet, um mindestens einen weiteren Injektionskanal mit Injektionssubstanz, insbesondere Zellen zu füllen.

In Figur 4 ist eine weitere Ausführungsform der erfindungsgemäßen Injektionseinrichtung 100 illustriert, die sich von der Variante gemäß Figur 1 insbesondere durch die Einstellung des Injektionswinkels unterscheidet. Gemäß Figur 4 bildet der Schwenkhebel 22 der Betätigungseinrichtung 20 einen Trägerarm zur Unterstützung der Injektionsspritze 50. Die Injektionsspritze 50 ist auf dem Schwenkhebel 22 verschiebbar angeordnet (siehe Doppelpfeil A in den Figuren 5, 6). Der Schwenkhebel 22 ist am Trägerteil 11 der Führungseinrichtung 10 verschwenkbar angelenkt. Durch eine Verschwenkung des Schwenkhebels 22 relativ zum Trägerarm 11.1 (siehe Doppelpfeil B in den Figuren 5, 6) kann der Injektionswinkel der Injektionsspritze eingestellt werden. Hierzu ist am Trägerteil 11 ein Klemmelement 18 vorgesehen, mit dem der Schwenkhebel 22 fixierbar ist. Diese Ausführungsform der Injektionseinrichtung 100 hat den Vorteil einer vereinfachten und besonders zuverlässigen Einstellung oder Nachjustierung des Injektionswinkels.

Mit der in Figur 4 gezeigten Ausführungsform wird der Injektionswinkel bei dem o. g. Kalibrierungsschritt mit dem Schwenkhebel 22 eingestellt, wobei das Betätigungselement 21 in einer zurückgeklappten Freigabeposition ist (gestrichelt gezeichnet). Für die Injektion wird nach dem Einsetzen der Injektionsspritze 50 in die Führungseinrichtung 10 und auf den Schwenkhebel 22 und dem Einstechen der Injektionsnadel in das Gewebe das Betätigungselement 21 vorgeschwenkt, um den Anschlag für den Injektionskolben 53 zu bilden. Durch den Rückzug der Injektionsnadel 51 und des Spritzenkörpers 52 (manuell oder mit einem Motorantrieb) erfolgt die Bildung des linienförmigen Injektionsvolumens im Gewebe.

Die Spritzennadel 51 kann im Trägerteil 11 mit einem Führungsrohr 12 oder einer Anschlagscheibe 12.1 geführt werden. Diese Varianten sind schematisch in den Figuren 5 und 6 illustriert. Das Führungsrohr 12 oder die Anschlagscheibe 12.1 übernehmen eine Doppelfunktion, nämlich erstens die Führung der Spritzennadel 51 und zweitens die Bildung eines vorderen Anschlags für den Spritzenkörper 52 der Injektionsspritze. Beim Einsetzen der Injektionsspritze in die Führungseinrichtung erfolgt ein Vorschub, bis der Spritzenkörper 52 am Führungsrohr 12 oder der Anschlagscheibe 12.1 anliegt.

Das Führungsrohr 12 oder die Anschlagscheibe 12.1 sind mit dem Schwenkhebel 22 fest verbunden und mit diesem in einem Spalt im Trägerteil 11 verschwenkbar.

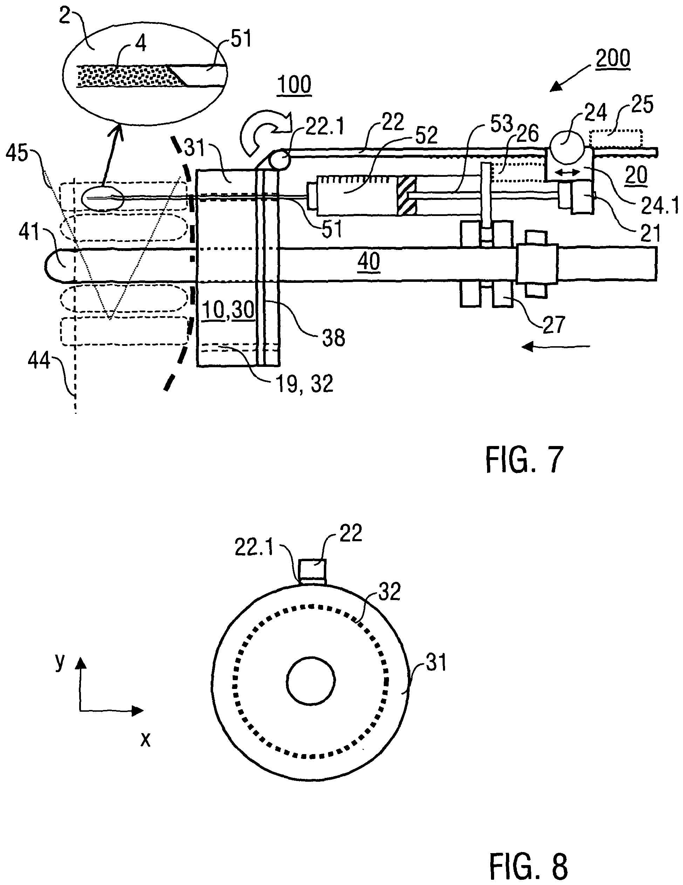

Die Figuren 7 und 8 illustrieren schematisch Merkmale von weiteren Ausführungsformen der erfindungsgemäßen Injektionseinrichtung 100, bei denen insbesondere vorgesehen ist, dass die Funktionen der Führungseinrichtung 10 und der Halteeinrichtung 30 gemeinsam durch die Halteplatte 31 erfüllt werden. Die Halteplatte 31 bildet eine Injektionsscheibe, die bei diesen Ausführungsformen eine Vielzahl von azimutal verteilten Durchgangsöffnungen 32 (siehe Vorderansicht in Figur 8) enthält, die jeweils einen Führungskanal 19 zur Aufnahme der Injektionsnadel 51 bilden. Abweichend von Figur 7 können die Durchgangsöffnungen radial verteilt angeordnet sein. Die Halteplatte 31 bildet auch eine Aufnahme für die Ultraschallsonde 40, deren Ultraschallkopf 41 eine transversale Schallebene 44 und eine longitudinale Schallebene 45 aufweist.

Figur 7 zeigt die Injektionsspritze während der Rückzugbewegung. Im vergrößerten Teilbild in Figur 7 ist die Bildung eines Injektionsdepots 4 mit einem linienförmigen Injektionsvolumen im externen Sphinkter 2 im Injektionskanal der zurückgezogenen Injektionsnadel 51 illustriert. Das Injektionsdepot 4 enthält z. B. Myoblasten, insbesondere autologe Myoblasten, die vom Organismus 1 gewonnen wurden. Das Injektionsdepot 4 hat z. B. einen Durchmesser im Bereich von 200 µm bis 1 mm und eine Länge im Bereich von 5 mm bis 10 cm.

Die Betätigungseinrichtung 20 umfasst den Schwenkhebel 22, der mit einem Gelenk 22.1 verschwenkbar an der Halteplatte 31 angeordnet ist. Das Gelenk 22.1 kann in einer Ringführung 38 auf einer Außenseite der Halteplatte 31 bewegt werden, um den Schwenkhebel 22 in Bezug auf eine der Durchgangsöffnungen 32 auszurichten. Der Schwenkhebel 22 trägt ein Translationselement 24 mit einem am Schwenkhebel 22 verschiebbaren Schlitten 24.1. Das Betätigungselement 21 ist am Schlitten 24.1 befestigt.

Der Schwenkhebel 22 kann in eine Freigabeposition verschwenkt werden (nicht dargestellt), in der die Injektionsspritze 50 in die Halteplatte 31 einsetzbar ist. Zur Injektion wird der Schwenkhebel 22 in eine Anschlagposition geschwenkt, in der das Betätigungselement 21 am Spritzenkolben 53 anliegt. In diesem Zustand erfolgen die Rückzugbewegung der Injektionsspritze 50 und die Injektion in das Gewebe. Bei der Injektion wird die Injektionsspritze 50 allein oder gemeinsam mit der Ultraschallsonde 40 von der ortsfesten Halteplatte 31 zurückgezogen.

Gemäß einer Variante der Erfindung kann auf das Gelenk 22.1 verzichtet werden. In diesem Fall ist der Schwenkhebel 22 in der Ringführung 38 verschiebbar angebracht, ohne in radialer Richtung verschwenkbar zu sein. Die Einstellung der Freigabeposition und der Anschlagposition erfolgt dann durch eine Translation des Schlittens 24.1.

Es kann vorgesehen sein, dass die Quantität der in das Gewebe eingeführten Injektionssubstanz unabhängig von der Geschwindigkeit der Rückzugbewegung eingestellt wird. Hierzu ist während der Rückzugbewegung der Injektionsnadel 51 eine axiale Verschiebung des Betätigungselements 21 relativ zu der Führungseinrichtung 10 vorgesehen. Die axiale Verschiebung des Betätigungselements 21 erfolgt z. B. mit einem Motorantrieb 25 oder einem Übertragungsmechanismus 26.

Mit dem Motorantrieb 25 kann das Betätigungselement 21 entgegengesetzt zur Rückzugbewegung verschoben werden. In diesem Fall kann die Injektion verstärkt werden, da die Substanzmenge, die je Weglänge injiziert wird, größer ist als mit einem ruhenden Betätigungselement 21. Mit dem Übertragungsmechanismus 26 kann das Betätigungselement 21 gleichsinnig mit der Rückzugbewegung verschoben werden, dies jedoch mit einer Geschwindigkeit, die kleiner als die Geschwindigkeit der Rückzugbewegung der Injektionsnadel 51 und des Spritzenkörpers 52 ist. In diesem Fall wird die je Weglänge injizierte Substanzmenge vermindert. Mit den Komponenten 25, 26 kann somit eine Übersetzung realisiert werden, durch die die injizierte Zellmenge pro mm Rückzugsstrecke nach Bedarf erhöht oder erniedrigt wird.

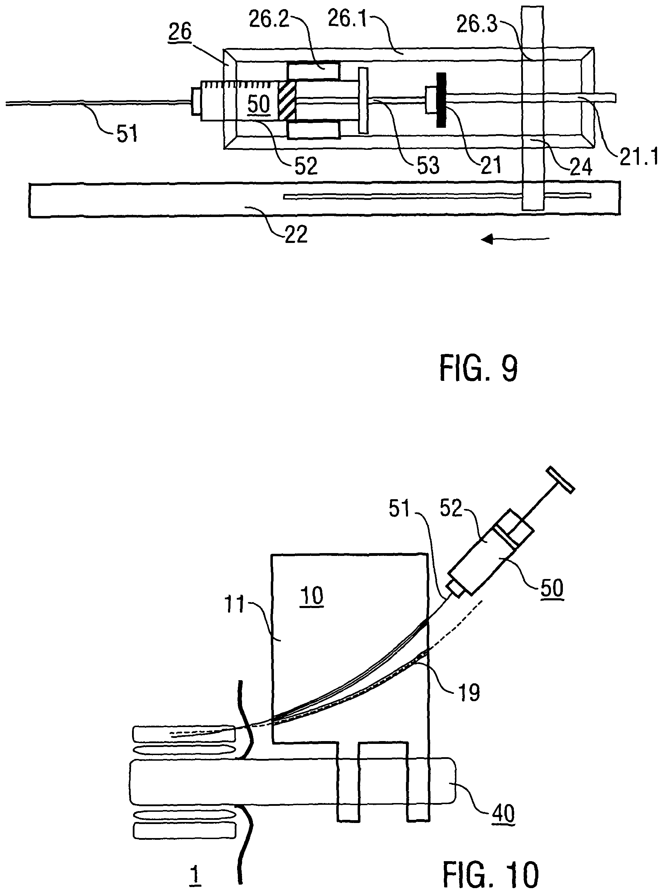

Der Übertragungsmechanismus 26 bildet eine Verbindung zwischen dem Spritzenkörper 52 und dem Betätigungselement 21, insbesondere mit dem Schlitten 24.1 des Translationselements 24. Der Übertragungsmechanismus 26 enthält eine Übersetzungsmechanik, wie z. B. einen Hebelmechanismus oder ein Schubgetriebe, mit der die Länge des Übertragungsmechanismus 26 in axialer Richtung veränderlich ist. Bei der Rückzugbewegung der Injektionsspritze 50 verschiebt der Spritzenkörper 52 über den Übertragungsmechanismus 26 auch den Schlitten 24.1 mit dem Betätigungselement 21. Die Übersetzungsmechanik ist dazu eingerichtet, dass die Geschwindigkeit des Betätigungselements 21 geringer als die Geschwindigkeit des Spritzenkörpers 52 ist. Eine Variante des Übertragungsmechanismus 26 ist in Figur 9 illustriert.

Typischerweise wird die Rückzugbewegung des Spritzenkörpers 52 manuell bewirkt. Alternativ kann gemäß einer weiteren Variante der Erfindung ein Motorantrieb 27 zur Bewegung des Spritzenkörpers 52 und der Spritzennadel 51 vorgesehen sein, der schematisch in Figur 7 gezeigt ist.

Figur 9 zeigt eine schematische Schnittansicht einer Ausführungsform eines erfindungsgemäß verwendeten Übertragungsmechanismus 26, der ein Hülsenteil 26.1 und ein Verbindungsstück 26.2 aufweist. Das Hülsenteil 26.1 ist über ein Schraubgewinde 26.3 mit dem Translationselement 24 verbunden, das am Schwenkhebel 22 (siehe auch Figuren 1, 4, 7) verschiebbar angeordnet ist. Der Spritzenkörper 52 ist mit dem Verbindungsstück 26.2 im Hülsenteil 26.1 fixiert. Das Betätigungselement 21 ist über einen Gewindestab 21.1, der durch das Translationselement 24 ragt und gegen Verdrehung gesichert ist, mit dem Hülsenteil 26.1 verbunden. Das Schraubgewinde 26.3 hat eine größere Gewindesteigung als der Gewindestab 21.1. Durch die Drehung des Hülsenteils 26.1 kann der Spritzenkörper 52 mit der Spritzennadel 51 zurückgezogen werden, während gleichzeitig der Gewindestab 21.1 und das Betätigungselement 21 mit geringerer Geschwindigkeit zurückgezogen werden.

In Figur 10 ist eine weitere Ausführungsform der erfindungsgemäß verwendeten Führungseinrichtung 10 schematisch illustriert (ohne die Betätigungseinrichtung), bei der im Trägerteil 11 ein oder mehrere gekrümmte Führungskanäle 19 vorgesehen sind. Bei dieser Ausführungsform wird der Injektionswinkel eingestellt, indem die Injektionsnadel 51 der Injektionsspritze 50 durch einen der Führungskanäle 19 mit der gewünschten Ausrichtung relativ zum Gewebe vorgeschoben wird.

Gemäß einer abgewandelten Variante der in Figur 10 gezeigten Ausführungsform der Erfindung kann im Trägerteil 11 mindestens ein gerader Führungskanal vorgesehen sein. Mit dem geraden Führungskanal wird ebenfalls der Injektionswinkel fest vorgegeben. Der Führungskanal übernimmt die Funktion der Führung der Injektionsnadel 51 und der Bildung eines Anschlags für den Spritzenkörper 52.

Im Folgenden wird die bevorzugte Anwendung der Erfindung bei der Behandlung der Stuhlinkontinenz beschrieben. Die erfindungsgemäße Injektionseinrichtung wird vorzugsweise zur Behandlung der Stuhlinkontinenz mit lebenden Muskelzellen verwendet. Durch die Injektion von gezüchteten Muskelzellen in den Schließmuskel des Darms wird die Kontraktionskraft gesteigert und somit Kontinenz wieder erzielt. Wenn die Behandlung besonders bevorzugt mit autologen Zellen erfolgt, sind die folgenden Schritte vorgesehen: (a) Muskelbiopsie, (b) Zellisolierung, (c) Kultivierung von Muskelzellen, insbesondere Myoblasten (d) Bildung der Injektionssubstanz und (e) Injektion in den Schließmuskel.

Bei den Schritten (a) bis (c) werden Myoblasten gezüchtet. Dabei werden an sich bekannte Biopsie- und Kultivierungstechniken angewendet. An welchem Muskel die Biopsie erfolgt, ist unkritisch. Aus der Probe (0,5 ... 2 g) werden Muskelstammzellen isoliert und vermehrt (einige hundert Millionen). Die Zellen werden entweder direkt in Spritzen verpackt oder als Zellpellet an den Behandlungsort gebracht. Bei Schritt (d) wird eine Suspension der Skelettmuskelzellen in einem Kultivierungsmedium gebildet. Das Kultivierungsmedium umfasst z. B. eine physiologische Lösung (z. B. mit Kochsalz, Phosphatbuffer), ein Zellkulturmedium mit autologem Serum. Die Suspension enthält z. B. 10 Millionen Zellen pro Milliliter. Anschließend erfolgt die Abfüllung in den Spritzenkörper der Injektionsspritze.

Alternativ ist möglich, dass die Bildung der Suspension der Skelettmuskelzellen mit Hilfe einer Doppelspritze mit zwei Spritzenkammern erfolgt. In einer Spritzenkammer werden die Zellen angeordnet, während in der anderen Spritzenkammer ein Medium angeordnet wird, das geeignet ist, zusammen mit den Zellen ein Gel zu bilden. Ein derartiges Medium umfasst z. B. autologes Plasma. Das im Plasma enthaltene Fibrin kann mit Kalzium ein Gel bilden. Bei der gleichzeitigen Injektion aus beiden Spritzenkammern erfolgt eine Durchmischung des Mediums mit den Zellen. Vorteilhafterweise werden damit die Zellen im Injektionskanal als Gelpolster eingebettet. Damit würde die Gefahr einer Ausspülung verhindert werden. Die Myoblasten lösen würden das Fibrin-Gel wieder auflösen, so dass keine störenden Effekte auf die Fasernbildung auftreten.

Die beladene Injektionsspritze wird in die Injektionseinrichtung eingesetzt, wobei die Injektionsnadel unter Ultraschallkontrolle z. B. in den externen Schließmuskel eingeführt wird, ohne andere Schichten des Enddarms zu verletzen. Der Einstich erfolgt vorzugsweise mit einem Abstand vom Anus von mindestens 1 cm, um eine Gefahr durch Keime zu reduzieren. Bei der Behandlung werden insgesamt z. B. 100 Millionen Zellen in mehreren Depots oder Bändern appliziert. Aus diesem Grund erfolgt die Zelltherapie am externen Sphinkter.

Alternativ oder zusätzlich kann eine Injektion von glatten Muskelzellen oder Vorläuferzellen, die sich in glatte Muskelzellen differenzieren, in den internen Sphinkter erfolgen.

Die in der vorstehenden Beschreibung, den Zeichnungen und den Ansprüchen offenbarten Merkmale der Erfindung können einzeln oder in Kombination für die Verwirklichung der Erfindung in ihren verschiedenen Ausgestaltungen von Bedeutung sein.

## Patentansprüche

1. Injektionseinrichtung (100), die zur Injektion mit einer Injektionsspritze (50) in einen Organismus (1) vorgesehen ist, umfassend:
- eine Führungseinrichtung (10), die zur Aufnahme der Injektionsspritze (50) eingerichtet ist, wobei eine Injektionsnadel (51) und ein Spritzenkörper (52) der Injektionsspritze (50) mit einer Vorschubbewegung zu oder einer Rückzugbewegung von dem Organismus (1) relativ zu der Führungseinrichtung (10) beweglich sind, und
- eine Betätigungseinrichtung (20), mit der die Injektionsspritze (50) koppelbar und die zur Betätigung der Injektionsspritze (50) für eine Injektion einer Injektionssubstanz in den Organismus (1) eingerichtet ist, wobei
- die Betätigungseinrichtung (20) ein Betätigungselement (21) aufweist, das einen Anschlag für eine Kolbeneinrichtung (53) der Injektionsspritze (50) an der Führungseinrichtung (10) derart bildet, dass die Kolbeneinrichtung (53) während der Rückzugbewegung eine relativ zu der Injektionsnadel (51) und dem Spritzenkörper (52) entgegengesetzte Relativbewegung ausführt, wobei das Betätigungselement (21) zur Freigabe der Kolbeneinrichtung (53) während der Vorschubbewegung der Injektionsnadel (51) eingerichtet ist,
**dadurch gekennzeichnet, dass**
- die Führungseinrichtung (10) ein Trägerteil (11) und ein Führungsrohr (12) zur Aufnahme der Injektionsnadel (51) aufweist, wobei das Führungsrohr (12) zur Einstellung eines Injektionswinkels der Injektionsspritze (50) am Trägerteil (11) verschwenkbar angeordnet ist, und
- mit der Führungseinrichtung (10) und/oder der Halteeinrichtung (30) eine bildgebende Sonde (40) verbunden ist.

2. Injektionseinrichtung nach Anspruch 1, bei der
- das Betätigungselement (21) verschwenkbar an der Stelleinrichtung (22, 24) angeordnet ist.

3. Injektionseinrichtung nach Anspruch 1 oder 2, bei der
- die Betätigungseinrichtung (20) eine Stelleinrichtung (22, 24) aufweist, mit der das Betätigungselement (21) an der Führungseinrichtung (10) angeordnet ist.

4. Injektionseinrichtung nach Anspruch 3, bei der
- die Stelleinrichtung einen Schwenkhebel (22) zur Ausrichtung des Betätigungselements (21) relativ zu der Führungseinrichtung (10) und/oder ein Translationselement (24) zur Verschiebung des Betätigungselements (21) relativ zu der Führungseinrichtung (10) aufweist.

5. Injektionseinrichtung nach mindestens einem der vorhergehenden Ansprüche, bei der
- die Ausrichtung der Injektionsspritze (50) mit einem Schwenkhebel (22) der Betätigungseinrichtung (20) einstellbar ist, wobei am Schwenkhebel (22) ein Führungsrohr (12) oder eine Anschlagscheibe (12.1) zur Führung der Injektionsnadel (51) befestigt ist.

6. Injektionseinrichtung nach mindestens einem der vorhergehenden Ansprüche 5, bei der
- das Führungsrohr (12) mit einem Drehlager (13) und einem Translationslager (14) am Trägerteil (11) angeordnet ist.

7. Injektionseinrichtung nach mindestens einem der vorhergehenden Ansprüche, die umfasst:
- eine Halteeinrichtung (30) mit einer Halteplatte (31), die auf den Organismus (1) aufsetzbar ist und sich entlang einer radialen Bezugsebene erstreckt, wobei die Führungseinrichtung (10) mit der Halteplatte (31) verbunden ist und die Halteplatte (31) mindestens eine Durchgangsöffnung (32) zum Durchtritt der Injektionsnadel (51) aufweist.

8. Injektionseinrichtung nach Anspruch 7, bei der
- die Halteeinrichtung (30) einen Halterahmen (33) aufweist, in dem die Halteplatte (31) drehbar angeordnet ist, und/oder
- die Halteplatte (31) eine Vielzahl von Durchgangsöffnungen (32) aufweist, die jeweils einen Führungskanal (19) der Führungseinrichtung (10) zur Aufnahme der Injektionsnadel (51) bilden.

9. Injektionseinrichtung nach Anspruch 7, bei der
- die Halteeinrichtung (30) einen sich axial erstreckenden Haltearm (35) aufweist, der mit der Halteplatte (31) verbunden ist und auf dem die bildgebende Sonde (40) verschiebbar angeordnet ist.

10. Medizinisches Gerät (200), das zur Injektion einer Injektionssubstanz in einen Organismus (1) eingerichtet ist, umfassend:
- eine Injektionseinrichtung (100) nach mindestens einem der vorhergehenden Ansprüche, und
- eine Injektionsspritze (50) mit einer Injektionsnadel (51), einem Spritzenkörper (52) und einer Kolbeneinrichtung (53), wobei die Injektionsspritze (50) in der Führungseinrichtung (10) der Injektionseinrichtung (100) angeordnet ist.

11. Medizinisches Gerät nach Anspruch 10, bei der
- die bildgebende Sonde (40) auf dem Haltearm (35) der Halteeinrichtung (30) der Injektionseinrichtung (100) verschiebbar angeordnet ist, und
- die Führungseinrichtung (10) an der bildgebenden Sonde (40) verschiebbar angeordnet ist.

## Claims

1. injection device (100), which is provided for injection with an injection syringe (50) into an organism (1), comprising:
- a guide device (10), which is adapted for receiving the injection syringe (50), an injection needle (51) and a syringe body (52) of the injection syringe (50) being movable with an advance movement towards or a withdrawal movement from the organism (1) relative to the guide device (10), and
- an actuating device (20), to which the injection syringe (50) can be coupled and which is adapted for actuation of the injection syringe (50) for an injection of an injection substance into the organism (1), wherein
- the actuating device (20) has an actuating element (21), which forms a limit stop for a piston device (53) of the injection syringe (50) on the guide device (10), so that during the with-drawal movement the piston device (53) performs an opposite movement relative to the injection needle (51) and the syringe body (52), wherein the actuating element (21) is adapted for release of the piston device (53) during the advance movement of the injection needle (51),
**characterized in that**
- the guide device (10) has a carrier part (11) and a guide tube (12) for receiving the injection needle (51), the guide tube (12) being arranged so that it can swivel on the carrier part (11) for setting an injection angle of the injection syringe (50), and
- an imaging probe (40) is connected to the guide device (10) and/or the holding device (30).

2. injection device according to claim 1, wherein
- the actuating element (21) is pivotably arranged on the positioning device (22, 24).

3. injection device according to claim 1 or 2, wherein
- the actuating device (20) has a positioning device (22, 24), with which the actuating element (21) is arranged on the guide device (10).

4. injection device according to claim 3, wherein
- the positioning device has a swivelling lever (22) for aligning the actuating element (21) relative to the guide device (10) and/or a translation element (24) for displacement of the actuating element (21) relative to the guide device (10).

5. injection device according to at least one of the preceding claims, wherein
- the orientation of the injection syringe (50) can be adjusted with a swivelling lever (22) of the actuating device (20), with a guide tube (12) or a stop ring (12.1) being secured on the swivelling lever (22) for guiding the injection needle (51).

6. injection device according to at least one of the preceding claims, wherein
- the guide tube (12) is arranged with a pivot bearing (13) and a translational bearing (14) on the carrier part (11).

7. injection device according to at least one of the preceding claims, which comprises:
- a holding device (30) with a holding plate (31), which can be placed on the organism (1) and extends along a radial reference plane, wherein the guide device (10) is connected to the holding plate (31) and the holding plate (31) has at least one through-hole (32) through which the injection needle (51) can pass.

8. injection device according to claim 7, wherein
- the holding device (30) has a holding frame (33), in which the holding plate (31) is arranged rotatably, and/or
- the holding plate (31) has a plurality of through-holes (32), each of which forms a guide channel (19) of the guide device (10) for receiving the injection needle (51).

9. injection device according to claim 7, wherein
- the holding device (30) has an axially extending holding arm (35), which is connected to the holding plate (31) and on which the imaging probe (40) is arranged displaceably.

10. Medical equipment (200), which is adapted for injection of an injection substance into an organism (1), comprising:
- an injection device (100) according to at least one of the preceding claims, and
- an injection syringe (50) with an injection needle (51), a syringe body (52) and a piston device (53), wherein the injection syringe (50) is arranged in the guide device (10) of the injection device (100).

11. Medical equipment according to claim 10, wherein
- the imaging probe (40) is arranged displaceably on the holding arm (35) of the holding device (30) of the injection device (100), and
- the guide device (10) is arranged displaceably at the imaging probe (40).

## Revendications

1. Dispositif d'injection (100), qui se destine à l'injection avec une seringue d'injection (50) dans un organisme (1), comprenant :
- un dispositif de guidage (10), qui est mis au point pour loger la seringue d'injection (50), sachant qu'une aiguille d'injection (51) et un corps de seringue (52) de la seringue d'injection (50) sont mobiles avec un déplacement d'avancement en direction de l'organisme (1) ou avec un déplacement de recul depuis l'organisme par rapport au dispositif de guidage (10), et
- un dispositif d'actionnement (20), auquel la seringue d'injection (50) peut être couplée et qui est mis au point pour l'actionnement de la seringue d'injection (50) en vue d'une injection d'une substance d'injection dans l'organisme (1), dans lequel
- le dispositif d'actionnement (20) présente un élément d'actionnement (21), qui forme une butée pour un dispositif de piston (53) de la seringue d'injection (50) au niveau du dispositif de guidage (10) de telle manière que le dispositif de piston (53) exécute, au cours du déplacement de recul, un déplacement relatif opposé par rapport à l'aiguille d'injection (51) et au corps de seringue (52), dans lequel l'élément d'actionnement (21) est mis au point pour libérer le dispositif de piston (53) au cours du déplacement d'avancement de l'aiguille d'injection (51),
**caractérisé en ce**
- **que** le dispositif de guidage (10) présente une partie de support (11) et un tuyau de guidage (12) servant à loger l'aiguille d'injection (51), dans lequel le tuyau de guidage (12) servant à régler un angle d'injection de la seringue d'injection (50) est disposé de manière à pouvoir pivoter au niveau de la partie de support (11), et
- en ce qu'une sonde d'imagerie (40) est reliée au dispositif de guidage (10) et/ou au dispositif de maintien (30).

2. Dispositif d'injection selon la revendication 1, dans le cadre duquel
- l'élément d'actionnement (21) est disposé de manière à pouvoir pivoter au niveau du dispositif de réglage (22, 24).

3. Dispositif d'injection selon la revendication 1 ou 2, dans le cadre duquel
- le dispositif d'actionnement (20) présente un dispositif de réglage (22, 24), à l'aide duquel l'élément d'actionnement (21) est disposé au niveau du dispositif de guidage (10).

4. Dispositif d'injection selon la revendication 3, dans le cadre duquel
- le dispositif de réglage présente un levier de pivotement (22) servant à orienter l'élément d'actionnement (21) par rapport au dispositif de guidage (10) et/ou un élément de translation (24) servant à faire coulisser l'élément d'actionnement (21) par rapport au dispositif de guidage (10).

5. Dispositif d'injection selon au moins l'une quelconque des revendications précédentes, dans le cadre duquel
- l'orientation de la seringue d'injection (50) peut être réglée avec un levier de pivotement (22) du dispositif d'actionnement (20), dans lequel un tuyau de guidage (12) ou un disque de butée (12.1) servant à guider l'aiguille d'injection (51) est fixé au niveau du levier de pivotement (22).

6. Dispositif d'injection selon au moins la revendication précédente 5, dans le cadre duquel
- le tuyau de guidage (12) est disposé par un palier rotatif (13) et un palier de translation (14) au niveau de la partie de support (11).

7. Dispositif d'injection selon au moins l'une quelconque des revendications précédentes, qui comprend :
- un dispositif de maintien (30) pourvu d'une plaque de maintien (31), qui peut être posée sur l'organisme (1) et qui s'étend le long d'un plan de référence radial, dans lequel le dispositif de guidage (10) est relié à la plaque de maintien (31) et la plaque de maintien (31) présente au moins une ouverture de passage (32) servant à faire passer l'aiguille d'injection (51).

8. Dispositif d'injection selon la revendication 7, dans le cadre duquel
- le dispositif de maintien (30) présente un cadre de maintien (33), dans lequel la plaque de maintien (31) est disposée de manière à pouvoir tourner, et/ou
- la plaque de maintien (31) présente une pluralité d'ouvertures de passage (32), qui forment respectivement un canal de guidage (19) du dispositif de guidage (10) servant à recevoir l'aiguille d'injection (51).

9. Dispositif d'injection selon la revendication 7, dans le cadre duquel
- le dispositif de maintien (30) présente un bras de maintien (35) s'étendant de manière axiale, qui est relié à la plaque de maintien (31) et sur lequel est disposée de manière à pouvoir coulisser la sonde d'imagerie (40).

10. Appareil médical (200), qui est mis au point pour la configuration d'une substance d'injection dans un organisme (1), comprenant :
- un dispositif d'injection (100) selon au moins l'une quelconque des revendications précédentes, et
- une seringue d'injection (50) pourvue d'une aiguille d'injection (51), d'un corps d'injection (52) et d'un dispositif de piston (53), dans lequel la seringue d'injection (50) est disposée dans le dispositif de guidage (10) du dispositif d'injection (100) .

11. Appareil médical selon la revendication 10, dans le cadre duquel
- la sonde d'imagerie (40) est disposée de manière à pouvoir coulisser sur le bras de maintien (35) du dispositif de maintien (30) du dispositif d'injection (100), et en ce
- que le dispositif de guidage (10) est disposé de manière à pouvoir coulisser au niveau de la sonde d'imagerie (40).
